(19) Europäisches Patentamt
European Patent Office
Office européen des brevets

(11) Publication number: 0 040 444 B1

(12) **EUROPEAN PATENT SPECIFICATION**

(45) Date of publication of patent specification: 21.03.84

(51) Int. Cl.[3]: C 01 B 33/28

(21) Application number: 81200465.3

(22) Date of filing: 01.05.81

(54) **Process for the preparation of crystalline silicates, crystalline silicates so prepared and process for carrying out catalytic reactions.**

(30) Priority: 16.05.80 NL 8002832

(43) Date of publication of application: 25.11.81 Bulletin 81/47

(45) Publication of the grant of the patent: 21.03.84 Bulletin 84/12

(84) Designated Contracting States: BE DE FR GB IT NL

(56) References cited:
EP - A - 0 018 090
DE - A - 2 817 576
US - A - 3 702 886
US - A - 3 760 024
US - A - 4 112 056

NATURE, Vol. 285, 1 May 1980 D.M. BIBBY et al. "NH4+-tetraalkyl Ammonium Systems in the Synthesis of Zeolites" pages 30 to 31

(73) Proprietor: SHELL INTERNATIONALE RESEARCH MAATSCHAPPIJ B.V.
Carel van Bylandtlaan 30
NL-2596 HR Den Haag (NL)

(72) Inventor: Boersma, Michael Adriaan Maria
Badhuisweg 3
NL-1031 CM Amsterdam (NL)
Inventor: Post, Martin Franciscus Maria
Badhuisweg 3
NL-1031 CM Amsterdam (NL)

(74) Representative: Puister, Antonius Tonnis, Mr. et al,
P.O. Box 302
NL-2501 CH The Hague (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 444 B1

# Process for the preparation of crystalline silicates, crystalline silicates so prepared and process for carrying out catalytic reactions

The invention relates to a process for the preparation of crystalline aluminium silicates having improved catalytic properties.

Mixtures of carbon monoxide and hydrogen can be converted into aromatic hydrocarbon mixtures with the use of a mixture of two catalysts, one of which is capable of catalyzing the conversion of an $H_2/CO$ mixture into acyclic oxygen-containing hydrocarbon derivatives and the other being a crystalline aluminium silicate capable of catalyzing the conversion of acyclic oxygen-containing hydrocarbon derivatives into aromatic hydrocarbons. The said crystalline aluminium silicates are characterized in that they have the following properties after one hour's calcination in air at 500°C:

a) thermally stable to a temperature above 600°C;
b) an X-ray powder diffraction pattern containing the four lines stated in Table A as strongest lines, and
c) in the formula representing the composition of the silicate expressed in moles of oxides, the $SiO_2/Al_2O_3$ molar ratio (for brevity designated m in the present patent application) is more than 10.

TABLE A

| d(Å) | Relative intensity |
|---|---|
| 11.1±0.2 | VS |
| 10.0±0.2 | VS |
| 3.84±0.07 | S |
| 3.72±0.06 | S |

where the letters used have the following meanings:

VS=very strong;
S=strong.

In European patent application No. 18 090 a zeolite product constituting ZSM-5/ZSM-11 intermediates is described. The X-ray diffraction pattern of the product contains as strongest lines the lines mentioned in Table A. The $SiO_2/Al_2O_3$ molar ratio of the product is preferably 150—3000. Said zeolite may be used as catalyst in the conversion of organic compounds, e.g. the aromatization of olefins and paraffins.

The relevant silicates can be prepared from an aqueous mixture containing the following compounds: one or more compounds of an alkali metal (M), one or more quaternary alkyl ammonium compounds ($R_4NX$), one or more silicon compounds with a high $SiO_2$ content and one or more aluminium compounds. In the present patent application by silicon compounds with a high $SiO_2$ content are meant: silicon compounds that yield a product with an $SiO_2$ content of more than 90% by weight after being dried at 120°C and calcined at 500°C. The crystallized silicates are prepared by keeping the aqueous mixture at elevated temperature until the crystalline silicate is formed, separating it from the mother liquor and calcining it. In the aqueous mixture from which the silicates are prepared, the various compounds should be present in the following ratios, expressed in moles of oxides:

$Na_2O:SiO_2<0.35$
$(R_2N)_2O:SiO_2<0.40$
$SiO_2:Al_2O_3>10$
$H_2O:SiO_2<65$

In an investigation by the Applicant into the use of the above-mentioned catalyst mixtures for the preparation of aromatic hydrocarbon mixtures starting from $H_2/CO$ mixtures with an $H_2/CO$ molar ratio below 1.0, it has been found that the $C_5^+$ (hydrocarbons with 5 or more carbon atoms) selectivity of said catalyst mixtures is greatly determined by the $SiO_2/Al_2O_3$ molar ratio of the crystalline silicate component. It has been found that on introducing into the catalyst mixture a crystalline silicate prepared with the application of an $Na_2O/SiO_2$ molar ratio of at most 0.04 in the aqueous base mixture usual for the preparation of said silicates, the $C_5^+$ selectivity of the catalyst mixtures rises accordingly when a crystalline silicate having a higher $SiO_2/Al_2O_3$ molar ratio is used and that for obtaining a $C_5^+$ selectivity of the catalyst mixtures that is acceptable for commercial use, the $SiO_2/Al_2O_3$ molar ratio of the crystalline silicate component must be more than 200. A drawback to the use of catalyst mixtures into which a crystalline silicate component is incorporated that is prepared with the application of the above-mentioned $Na_2O/SiO_2$ molar ratio in the aqueous base mixture is that the activity of said catalyst mixtures decreases accordingly when a crystalline silicate with a higher $SiO_2/Al_2O_3$ molar ratio is used. Further investigation by the Applicant into said subject showed that said catalyst mixtures exhibit both a high activity and a high $C_5^+$ selectivity if they contain a crystalline silicate component with an

$SiO_2/Al_2O_3$ molar ratio of more than 300 that is prepared with the application of an $Na_2O/SiO_2$ molar ratio in the aqueous base mixture of 0.08—0.16. The preparation of crystalline aluminium silicates with an $SiO_2/Al_2O_3$ molar ratio of more than 300 from an aqueous mixture in which the $Na_2O/SiO_2$ molar ratio has the above-mentioned high value, is novel.

The present patent application therefore relates to a process for the preparation of crystalline aluminium silicates that after one hour's calcination in air have the properties stated under a) and b) and a value of more than 300 for m as meant under c), in which an aqueous mixture containing the following compounds: one or more compounds of an alkali metal (M), one or more quaternary alkyl ammonium compounds ($R_4NX$), one or more silicon compounds with a high $SiO_2$ content and one or more aluminium compounds, in which mixture the various compounds are present in the following ratios, expressed in moles of oxides:

$M_2O:SiO_2=0.08—0.16$
$(R_4N)_2O:SiO_2=0.01—0.40$
$H_2O:SiO_2=5—65$, and
$SiO_2:Al_2O_3>400$,

is maintained at elevated temperature until the crystalline silicate is formed which is subsequently separated from the mother liquor, dried and calcined.

The silicates prepared according to the invention are inter alia defined with reference to the X-ray powder diffraction pattern. This pattern should contain the four lines stated in Table A as strongest lines. The complete X-ray powder diffraction pattern of a typical example of a silicate prepared according to the invention is stated in Table B:

TABLE B

| d(Å) | Relative intensity | d(Å) | Relative intensity |
|---|---|---|---|
| 11.1 | 100 | 4.00 | 3 |
| 10.0 | 70 | 3.84 | 57 |
| 8.93 | 1 | 3.72 | 31 |
| 7.99 | 1 | 3.64 | 10 |
| 7.42 | 1 | 3.44 | 5 |
| 6.68 | 7 | 3.34 | 3 |
| 6.35 | 11 | 3.30 | 5 |
| 5.97 | 18 | 3.25 | 2 |
| 5.70 | 7 | 3.05 | 5 |
| 5.56 | 10 | 2.98 | 12 |
| 5.35 | 2 | 2.96 | 3 |
| 4.98 | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |

The silicates can be prepared both at atmospheric pressure and at elevated pressure. If use is made of reaction temperatures lying above the boiling point of the mixture it is preferred to work under autogenous pressure in an autoclave. The silicates are preferably prepared by maintaining the mixture for at least four hours at a temperature between 90 and 300°C and in particular between 125 and 175°C. After formation of the silicates the crystals are separated from the mother liquor, for example by means of filtration, decantation and centrifuging. The crystal mass is subsequently washed with water and finally dried and calcined.

Examples of suitable compounds which can be used in the preparation of the silicates according to the invention are nitrates, carbonates, hydroxides and oxides of alkali metals; quaternary alkyl ammonium bromides and hydroxides; amorphous solid silicas, silicasols, silica gels and silicic acid; aluminium hydroxide, sodium aluminate, aluminium sulphate and gamma-alumina. In the preparation of the silicates according to the invention it is preferred to start from an aqueous mixture in which M is present in a sodium compound and $R_4NX$ is a tetrapropyl ammonium compound.

Silicates prepared according to the invention can inter alia be used as adsorbent and extractant means, drying agent, ion-exchanger and as catalyst or catalyst carrier in the operation of various catalytic processes, in particular the catalytic preparation of aromatic hydrocarbons from acyclic organic compounds. If the intention is to use the silicates prepared according to the invention as catalyst or catalysts carrier, it is preferred to reduce the alkali metal content of these silicates previously to less than 0.1% by weight and in particular to less than 0.01% by weight. The reduction of the alkali metal content of the silicates can very suitably be carried out by contacting the silicates once or several times with an aqueous solution containing ammonium ions. From the $NH_4^+$ silicates thus obtained the

$H^+$ silicates can be prepared by calcination. When the crystalline aluminium silicates are used as catalyst they can be combined, if desired, with a binder material, such as bentonite or kaolin.

As explained in the foregoing an important application of the silicates prepared according to the invention is their use in catalyst mixtures for the preparation of an aromatic hydrocarbon mixture from an $H_2/CO$ mixture having an $H_2/CO$ molar ratio below 1.0. Such $H_2/CO$ mixtures can very suitably be prepared by steam gasification of coal at a temperature between 900 and 1500°C and a pressure between 10 and 50 bar. The preparation of the aromatic hydrocarbon mixture from an $H_2/CO$ mixture with an $H_2/CO$ molar ratio below 1.0 with the use of a catalyst mixture containing a crystalline aluminium silicate prepared according to the invention, is preferably carried out at a temperature of 200—500°C and in particular of 300—450°C, a pressure of 1—150 bar and in particular of 5—100 bar and a space velocity of 50—5000 and in particular of 300—3000 Nl of gas/l of catalyst/h. The two catalysts present in the catalyst mixture used in the preparation of an aromatic hydrocarbon mixture from an $H_2/CO$ mixture having an $H_2/CO$ molar ratio below 1.0 will for brevity hereinafter be designated catalysts X and Y. Catalyst X is the one having the capacity of catalyzing the conversion of an $H_2/CO$ mixture into acyclic oxygen-containing hydrocarbon derivatives and catalyst Y is the crystalline aluminium silicate prepared according to the invention. As catalyst X it is preferred to use catalysts capable of converting an $H_2/CO$ mixture into substantially methanol and/or dimethyl ether. If the aim is to prepare a product mainly consisting of hydrocarbons boiling in the gasoline range, as catalyst X use can very suitably be made of a catalyst containing zinc together with chromium. When such a catalyst is used, preference is given to a catalyst in which the atomic percentage of zinc, based on the sum of zinc and chromium, is at least 60% and in particular 60—80%. Further, it is preferred to use mixtures containing 1—5 volume parts of catalyst X per volume part of catalyst Y.

The conversion of an $H_2/CO$ mixture with an $H_2/CO$ molar ratio below 1.0 into an aromatic hydrocarbon mixture may suitably be carried out in two stages. In the first stage the $H_2/CO$ mixture is passed over a mixture of a crystalline aluminium silicate prepared according to the invention and of a catalyst capable of catalyzing the conversion of an $H_2/CO$ mixture into acyclic oxygen-containing hydrocarbon derivatives. The reaction product of the first stage or at least its $C_2^-$ fraction, i.e. the fraction containing hydrocarbons or oxygen-containing hydrocarbon derivatives with 1 or 2 carbon atoms and $H_2$, $CO$, $CO_2$ and $H_2O$, is contacted in a second stage with a catalyst containing one or more metal components with catalytic activity for the conversion of an $H_2/CO$ mixture into acyclic hydrocarbons, which metal components are chosen from the group consisting of cobalt, nickel and ruthenium. If the feed for the second stage has an $H_2/CO$ molar ratio of less than 1.5, water is added to said feed. Then in the second stage a bi-functional catalyst combination is used containing, in addition to the metal components having catalytic activity for the conversion of an $H_2/CO$ mixture into acyclic hydrocarbons, also one or more metal components with catalytic activity for the conversion of an $H_2O/CO$ mixture into an $H_2/CO_2$ mixture.

The invention is now illustrated with reference to the following Example.

Example

Six crystalline aluminium silicate (silicates 1—6) were prepared by heating mixtures of NaOH, amorphous silica, $(C_3H_7)_4NOH$ and $NaAlO_2$ in water at 150°C for 24 hours in an autoclave. After the reaction mixtures had been cooled the silicates obtained were filtered off, washed with water until the pH of the washing water was about 8, dried at 120°C and calcined at 500°C. The silicates 1—6 had the following properties:

a) thermally stable to a temperature above 800°C;
b) an X-ray powder diffraction pattern mainly corresponding with that stated in Table B, and
c) a value for m as stated in Table C.

TABLE C

| Silicate No. | m |
|---|---|
| 1 | 290 |
| 2 | 285 |
| 3 | 450 |
| 4 | 700 |
| 5 | 330 |
| 6 | 310 |

After drying at 120°C and calcining at 500°C the amorphous silica used in the preparation of silicates 1—6 yielded a product containing 99.97% by weight of $SiO_2$.

The molar composition of the aqueous mixtures from which silicates 1—6 were prepared can be represented as follows:

$$25\ SiO_2 \,.\, x\ Al_2O_3 \,.\, y\ Na_2O \,.\, 4.5[(C_3H_7)_4N]_2O \,.\, 450\ H_2O$$

where x and y have the values stated in Table D. The $Na_2O/SiO_2$ molar ratios used in the aqueous mixtures are also stated in Table D:

TABLE D

| Silicate No. | x | y | $Na_2O/SiO_2$ molar ratio |
|---|---|---|---|
| 1 | 0.063 | 1.0 | 0.04 |
| 2 | 0.039 | 3.0 | 0.12 |
| 3 | 0.042 | 1.0 | 0.04 |
| 4 | 0.015 | 3.0 | 0.12 |
| 5 | 0.056 | 1.0 | 0.04 |
| 6 | 0.031 | 3.0 | 0.12 |

From silicates 1—6 silicates I—VI were respectively prepared by boiling the materials calcined at 500°C with 1.0 molar $NH_4NO_3$ solution, washing with water, reboiling with a 1.0 molar $NH_4NO_3$ solution, washing and drying at 120°C and calcining at 500°C. Subsequently, six catalyst mixtures (A—F) were prepared by mixing a $ZnO—Cr_2O_3$ composition with each of the silicates I—VI. The atomic Zn-percentage of $ZnO—Cr_2O_3$ composition, based on the sum of Zn and Cr, was 70%. All the catalyst mixtures contained per weight part of silicate, 10 parts by weight of the $ZnO—Cr_2O_3$ composition. The catalyst mixtures A—F were tested for the preparation of an aromatic hydrocarbon mixture from an $H_2/CO$ mixture. The test was carried out in a 50 ml reactor containing a fixed catalyst bed having a volume of 7.5 ml. In six experiments an $H_2/CO$ mixture with an $H_2/CO$ molar ratio of 0.5 was passed over each of the catalyst mixtures A—F at a temperature of 375°C, a pressure of 60 bar and a space velocity of 1000 $Nl.l^{-1}.h^{-1}$. In all the cases a product was obtained, the $C_5^+$ fraction of which contained more than 50% by weight of aromatics. The remaining results of the experiments are stated in Table E:

TABLE E

| Exp. No. | Cat. mix No. | Silicate No. | $C_5^+$ selectivity after 10 hours, % by wt | Conversion of the syngas after 10 hours, % by vol. |
|---|---|---|---|---|
| 1 | A | I | 65 | 65 |
| 2 | B | II | 64 | 66 |
| 3 | C | III | 75 | 40 |
| 4 | D | IV | 80 | 56 |
| 5 | E | V | 75 | 48 |
| 6 | F | VI | 77 | 58 |

Of the silicates stated in Table D only silicates 4 and 6 were prepared according to the invention. Silicates 1, 2, 3 and 5 fall outside the scope of the invention. They are included in the patent application for comparison. Of the experiments stated in Table E, only experiments 4 and 6 were carried out with the use of a catalyst mixture containing a crystalline aluminium silicate prepared according to the invention. Experiments 1, 2, 3 and 5 fall outside the scope of the invention. They are included in the patent application for comparison.

It is apparent from the results stated in Table E that in the conversion of an $H_2/CO$ mixture with an $H_2/CO$ molar ratio below 1.0 into an aromatic hydrocarbon mixture with the use of a catalyst mixture containing a crystalline aluminium silicate with an $SiO_2/Al_2O_3$ molar ratio of less than 300, the activity of the catalyst mixture is independent of the $Na_2O/SiO_2$ molar ratio in the base mixture from which the relevant silicate was prepared. Further, it appears from the results that when use is made of a catalyst mixture containing a crystalline aluminium silicate having an $SiO_2/Al_2O_3$ molar ratio of more than 300, the activity of the catalyst mixture for said conversion is greatly dependent on said molar ratio in the base mixture and that by a correct choice of said molar ratio, catalyst mixtures can be prepared having a high activity in addition to a very high $C_5^+$ selectivity.

**Claims**

1. A process for the preparation of crystalline aluminium silicates having the following properties after one hour's calcination in air at 500°C:

a) thermally stable to a temperature above 600°C;
b) an X-ray powder diffraction pattern containing the four lines stated in Table A as strongest lines.

TABLE A

| d(Å) | Relative intensity |
|---|---|
| 11.1±0.2 | VS |
| 10.0±0.2 | VS |
| 3.84±0.07 | S |
| 3.72±0.06 | S |

where the letters used have the following meanings:
VS=very strong;
S=strong, and

c) in the formula representing the composition of the silicate expressed in moles of oxides, the $SiO_2/Al_2O_3$ molar ratio (m) is more than 300,

characterized in that an aqueous mixture containing the following compounds: one or more compounds of an alkali metal (M), one or more quaternary alkyl ammonium compounds ($R_4NX$), one or more silicon compounds that after drying at 120°C and calcination at 500°C yield a product with an $SiO_2$ content of more than 90% by weight, and one or more aluminium compounds in which mixture the various compounds are present in the following molar ratios, expressed in moles of oxides:

$M_2O:SiO_2=0.08—0.16$
$(R_4N)_2O:SiO_2=0.01—0.40$
$H_2O:SiO_2=5—65$, and
$SiO_2:Al_2O_3>400$,

is maintained at elevated temperature until the crystalline silicate is formed which is subsequently separated from the mother liquor, dried and calcined.

2. A process as claimed in claim 1, characterized in that as $R_4NX$ compound a tetrapropyl ammonium compound and as alkali metal compound a sodium compound is used.

3. A process as claimed in claim 1 or 2, characterized in that the mixture is kept at a temperature between 90 and 300°C for at least four hours.

4. A process as claimed in claim 3, characterized in that the mixture is kept at a temperature between 125 and 175°C.

5. Crystalline aluminium silicates prepared by a process as described in any one or more of claims 1—4, characterized in that the alkali metal content thereof is reduced to less than 0.1% by weight.

6. Crystalline aluminium silicates as claimed in claim 5, characterized in that the alkali metal content is reduced to less than 0.01% by weight.

7. Use of a crystalline aluminium silicate prepared according to any one or more of claims 1—4 in the preparation of an aromatic hydrocarbon mixture in which an $H_2/CO$ mixture having an $H_2/CO$ molar ratio below 1.0 is contacted with a mixture of two catalysts, one of which being capable of catalyzing the conversion of an $H_2/CO$ mixture into acyclic oxygen-containing hydrocarbon derivatives and the other being the crystalline aluminium silicate.

**Revendications**

1. Un procédé pour la préparation de silicates d'aluminium cristalline ayant les propriétés suivantes après calcination pendant une heure dans l'air à 500°C:

a) ils sont thermiquement stables jusqu'à une température de plus de 600°C;

b) ils ont un diagramme de diffraction des rayons X par la méthode des poudres contentant les quatre lignes spécifiées dans le Tableau A comme lignes les plus intenses:

TABLEAU A

| d(Å) | Intensité relative |
|---|---|
| 11,1±0,2 | VS |
| 10,0±0,2 | VS |
| 3,84±0,07 | S |
| 3,72±0,06 | S |

où les lettres utilisées ont les significations suivantes:
VS=très forte;
S=forte, et

c) dans la formule représentant la composition du silicate exprimée en moles des oxydes, le rapport molaire $SiO_2/Al_2O_3$ (m) est supérieur à 300,

caractérisé en ce qu'un mélange aqueux contenant les composés suivants: un ou plusieurs composés d'un métal alcalin (M), un ou plusieurs composés d'alcoylammonium quaternaire ($R_4NX$), un ou plusieurs composés du silicium qui aprés séchage à 120°C et calcination à 500°C donnent un produit d'une teneur en $SiO_2$ de plus de 90% en poids, et un ou plusieurs composés de l'aluminium, mélange dans lequel les divers composés sont présents dans les rapports molaires suivants, exprimés en moles des oxydes:

$M_2O:SiO_2$=0,08—0,16
$(R_4N)_2O:SiO_2$=0,01—0,40
$H_2O:SiO_2$=5—65, et
$SiO_2:Al_2O_3$>400,

est maintenu à température élevée jusqu'à ce que le silicate cristallin soir formé, et ce dernier est ensuite séparé de la liqueur-mère, séché et calciné.

2. Un procédé selon la revendication 1, caractérisé en ce que comme composé $R_4NX$ on utilise un composé de tétrapropylammonium et comme composé de métal alcalin un composé du sodium.

3. Un procédé selon la revendication 1 ou 2, caractérisé en ce que le mélange est maintenu à une température comprise entre 90 et 300°C pendant au moins quatre heures.

4. Un procédé selon la revendication 3, caractérisé en ce que le mélange est maintenu à une température comprise entre 125 et 175°C.

5. Des silicates d'aluminium cristallins préparés par un procédé tel que décrit dans une ou plusieurs quelconques des revendications 1 à 4, caractérisés en ce que leur teneur en métaux alcalins est réduite à moins de 0,1% en poids.

6. Des silicates d'aluminium crystallins selon la revendication 5, caractérisés en ce que leur teneur en métaux alcalins est réduite à moins de 0,01% en poids.

7. L'utilisation d'un silicate d'aluminium cristallin préparé selon une ou plusieurs quelconques des revendications 1 à 4 dans la préparation d'un mélange d'hydrocarbures aromatiques dans laquelle un mélange $H_2/CO$ ayant un rapport molaire $H_2/CO$ inférieur à 0,1 est mis en contact avec un mélange de deux catalyseurs, l'un d'entre eux étant capable de catalyser la transformation d'un mélange $H_2/CO$ en dérivés oxygénés d'hydrocarbures acycliques et l'autre étant le silicate d'aluminium cristallin.

**Patentansprüche**

1. Ein Verfahren zur Herstellung von kristallinen Aluminiumsilikaten, welche die nachstehenden Eigenschaften nach einstündiger Calcinierung in Luft bei 500°C aufweisen:

a) sie sind bis zu einer Temperatur oberhalb 600°C stabil;
b) ein Röntgenpulverdiagramm enthält die in Tabelle A angegebenen 4 Linien als stärkste Linien:

TABELLE A

| d(Å) | Relative Intensität |
|---|---|
| 11,1±0,2 | VS |
| 10,0±0,2 | VS |
| 3,84±0,07 | S |
| 3,72±0,06 | S |

in welcher die verwendeten Buchstaben die folgende Bedeutung haben:
VS=sehr stark,
S=stark, und

c) in der die Zusammensetzung des Silikats wiedergebenden Formel, ausgedrückt in Molen der Oxide, hat das molare Verhältnis (m) von $SiO_2/Al_2O_3$ einen Wert von mehr als 300,

dadurch gekennzeichnet, daß eine wässrige Mischung, welche die nachstehenden Verbindungen enthält: eine oder mehrere Verbindungen eines Alkalimetalls (M), ein oder mehrere quaternäre Alkylammoniumverbindungen ($R_4NX$), eine oder mehrere Siliciumverbindungen, welche nach dem Trocknen bei 120°C und Calcinieren bei 500°C ein Produkt mit einem $SiO_2$-Gehalt von mehr als 90 Gewichtsprozent ergeben, und ein oder mehrere Aluminiumverbindungen, in welcher Mischung die verschiedenen Verbindungen in den nachstehenden molaren Verhältnissen, ausgedrückt in Molen der Oxide, enthalten sind:

$M_2O:SiO_2$=0,08—0,16
$(R_4N)_2O:SiO_2$=0,01—0,40
$H_2O:SiO_2$=5—65, und
$SiO_2:Al_2O_3$>400

auf erhöhter Temperatur gehalten wird, bis das kristalline Silikat gebildet ist, welches anschließend aus der Mutterlauge abgetrennt, getrocknet und calciniert wird.

2. Ein Verfahren wie in Anspruch 1 beansprucht, dadurch gekennzeichnet, daß als Verbindung $R_4NX$ eine Tetrapropylammoniumverbindung und als Alkalimetallverbindung eine Natriumverbindung verwendet wird.

3. Ein Verfahren wie in Anspruch 1 oder 2 beansprucht, dadurch gekennzeichnet, daß die Mischung mindestens 4 Stunden lang auf einer Temperatur zwischen 90 und 300°C gehalten wird.

4. Ein Verfahren wie in Anspruch 3 beansprucht, dadurch gekennzeichnet, daß die Mischung auf einer Temperatur zwischen 125 und 175°C gehalten wird.

5. Kristalline Aluminiumsilikate, hergestellt durch ein Verfahren wie in irgendeinem der Ansprüche 1 bis 4 beschrieben, dadurch gekennzeichnet, daß der Alkalimetallgehalt auf unter 0,1 Gewichtsprozent verringert worden ist.

6. Kristalline Aluminiumsilikate, wie in Anspruch 5 beansprucht, dadurch gekennzeichnet, daß der Alkalimetallgehalt auf unter 0,01 Gewichtsprozent verringert worden ist.

7. Verwendung eines kristallinen Aluminiumsilikats, hergestellt gemäß irgendeinem der Ansprüche 1 bis 4, zur Herstellung einer Mischung von aromatischen Kohlenwasserstoffen, wobei eine $H_2$/CO-Mischung mit einem molaren Verhältnis an $H_2$/CO unterhalb 1,0 mit einer Mischung von 2 Katalysatoren kontaktiert wird, von denen einer in der Lage ist, die Umwandlung einer $H_2$/CO-Mischung in acyclische sauerstoffhaltige Kohlenwasserstoffderivate/katalysieren, und der andere das kristalline Aluminiumsilikat ist.